(19) Europäisches Patentamt
European Patent Office
Office européen des brevets

(11) Publication number: **0 605 828 A1**

(12) **EUROPEAN PATENT APPLICATION**

(21) Application number: **93120394.7**

(22) Date of filing: **17.12.93**

(51) Int. Cl.[5]: **C12Q 1/68**, C12P 19/34, G01N 33/543

(30) Priority: **04.01.93 US 425**

(43) Date of publication of application: **13.07.94 Bulletin 94/28**

(84) Designated Contracting States: **DE FR GB IT**

(71) Applicant: **BECTON, DICKINSON & COMPANY**
**One Becton Drive**
**Franklin Lakes New Jersey 07417-1880(US)**

(72) Inventor: **Feindt, Hans H.**
**9 Prettyboy Garth**
**Parkton, Maryland 21120(US)**
Inventor: **Mallonee, Richard L.**
**217 Oak Forest Avenue**
**Catonsville, Maryland 21228(US)**
Inventor: **McFarland, Edward C.**
**2605 Wendover Road**
**Parkville, Maryland 21120(US)**

(74) Representative: **Gerbino, Angelo et al**
**c/o JACOBACCI-CASETTA & PERANI S.p.A.**
**Via Alfieri 17**
**I-10121 Torino (IT)**

(54) **Flow-through hybridization assay for oligonucleotide sequences.**

(57) Flow-through nucleic acid hybridization assays in which ligand-derivatized probes are synthesized using a target nucleic acid sequence as the template. The probe is immobilized on the membrane of a flow-through assay device by binding to a specific binding pair member for the ligand or hybridization to a complementary nucleic acid sequence. The probe on the membrane of a flow-through assay device, indicating the template target sequence was present during probe synthesis, is detected by means of a tracer comprising a specific binding molecule for the ligand.

FIG-1

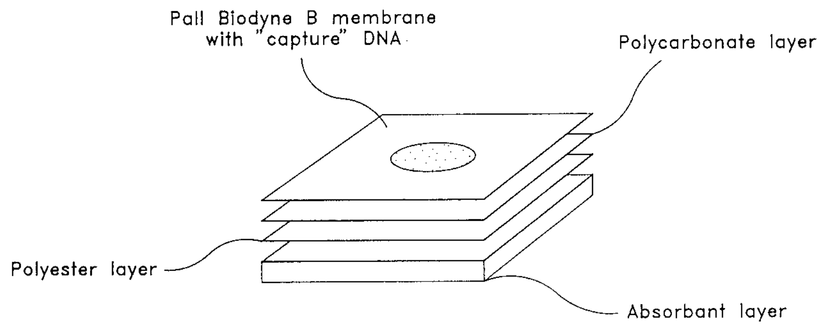


EP 0 605 828 A1

## FIELD OF THE INVENTION

The present invention relates to membrane flow-through assays for detecting nucleic acids.

## BACKGROUND OF THE INVENTION

Complementarity of nucleic acid sequences is a well known means for identifying, detecting and isolating specific nucleic acids and nucleic acid sequences. Such methods form the basis of many current nucleic acid assays, in particular hybridization assays. The principle of hybridization assays is to expose two or more single stranded nucleic acids to each other, allow complementary sequences to hybridize, and then measure the amount of double stranded nucleic acid formed. Alternatively, successful synthesis of a complementary nucleic acid strand can be used to indicate the presence of the template strand which directed its synthesis. The nucleic acid or sequence which is sought to be identified, detected or isolated is customarily called the target nucleic acid or target sequence.

A single stranded nucleic acid or sequence which is complementary to the target nucleic acid used to isolate, identify or detect the target is customarily called a probe. In some methods of nucleic acid hybridization, more than one probe may be used for different purposes. A probe often has a detectable label incorporated into the structure of the probe which facilitates identification of the target nucleic acid when the probe hybridizes to it. The label produces a signal which can be detected either visually or by instrumentation. Many of the nucleic acid assays known in the art depend on hybridization of complementary single strands. In solution hybridization procedures, the target nucleic acid and the probe(s) are brought together in solution and allowed to hybridize. Hybridization of the probe to the target can be followed by changes in optical density as double stranded nucleic acid is formed. Alternatively, double stranded nucleic acid may be separated from unreacted single strands and detected by means of a label present in the hybridized material. For example, hydroxyapatite can be used to selectively bind double stranded nucleic acids or $S_1$ nuclease can be used to specifically degrade unreacted single stranded nucleic acid, which can then be separated from hybridized double stranded nucleic acid by size separation methods known in the art.

Solution hybridization is rapid and allows for efficient reaction between complementary nucleic acid sequences. However, subsequent separation and detection of the hybridized products is labor intensive, time consuming and difficult to automate. In an attempt to overcome some of these drawbacks, solid-surface hybridization methods have been developed in which one of the single stranded components of the hybridization mixture is immobilized on a solid support. Hybridization of the immobilized nucleic acid with complementary single stranded sequences in a solution of the remaining assay components results in immobilization of the entire hybridized complex through base pairing. The solid support is then easily removed from the solution to separate hybridized from unhybridized components. The occurrence of hybridization can be determined directly on the solid surface by detection of the label present in the hybrid.

Solid surface or solid phase nucleic acid hybridization methods provide rapid and simple separation of hybridized nucleic acids from unhybridized single stranded nucleic acids in the assay. However, they also exhibit several drawbacks. The kinetics of hybridization on a solid surface where not all of the reaction components can diffuse result in significantly slower reaction rates. As a result, hybridization and washing steps take much longer when a solid support is involved and assay sensitivity is often reduced. In addition, binding of nucleic acids to a solid support makes some of the bases unavailable for pairing, further reducing hybridization efficiency.

In immunological assays, flow through solid phase methods have been developed to provide more rapid results without sacrificing the ease of reagent separation associated with traditional solid-phase assays. Some of the known immunological flow-through assays and the devices designed for use in such assays are described in U.S. Patent No. 4,366,241 to Tom, et al., U.S. Patent Nos. 4,727,019 and 4,632,901 to Valkirs, et al. and U.S. Patent No. 4,818,677 to Hay-Kaufman, et al. However, these immunological methods have not been fully adapted to nucleic acid hybridization assays, as the known flow-through nucleic acid hybridization assays require a period of incubation prior to initiating flow through the reaction surface in order for efficient hybridization to take place. This incubation period negates one of the major advantages of the flow-through assay format, i.e., the speed with which a result can be obtained.

WO 89/10979 discloses one such solid phase nucleic acid hybridization assay. In one embodiment, a sandwich hybridization is performed in solution and the hybrids are captured by a complementary capture ligand on a porous membrane in an assay format similar to the flow through method commonly used for immunoassays. However, to achieve sufficient binding the solution is incubated with the membrane for 10 min. prior to allowing the solution to flow through. The method also requires two probes for detection of the desired target nucleic acid - one carrying a label and a second carrying a ligand for capture.

While some time savings have been achieved by using a controlled or restricted flow-through sandwich nucleic acid assay as in WO 89/10979, none of the prior art methods have found a way to take full advantage of the speed and simplicity of flow-through membrane assays for nucleic acid hybridization. That is, the prior art has not successfully eliminated a period of incubation with the membrane while maintaining a satisfactory level of sensitivity in nucleic acid hybridization assays. The invention is distinguished from prior art flow-through nucleic acid hybridization assays in that fluid flow through the device is unrestricted, i.e., there is no period of incubation of assay components with the membrane during which flow-through is prevented to allow hybridization to occur.

Corti et al. (1990) J. Immunol. Mtds. 134: 81-86 describe a method for evaluating the efficiency of DNA hapten labelling reactions in which antihapten antibody/alkaline phosphatase conjugates are used to detect hapten-DNA immobilized on capillary absorbent filters. The hapten-DNA is directly absorbed onto the filter.

The present invention overcomes several problems in the art of solid-surface nucleic acid hybridization. A flow-through membrane hybridization assay is provided which requires no period of incubation of the sample with the membrane. The assay therefore allows rapid, simple detection of ligand-containing nucleic acids on a solid surface and is useful for screening nucleic acid amplification reaction products and for a variety of diagnostics based on detection of specific nucleic acid sequences.

## SUMMARY OF THE INVENTION

The present invention provides a flow-through assay method in which nucleic acid probes containing a ligand are used to detect the desired target nucleic acid sequence. In one embodiment, the probes are synthesized with incorporation of the ligand using the target sequence as a template. The presence of the synthesized ligand-containing nucleic acid probe is then detected directly as an indication of the presence of the target by binding to an anti-ligand antibody or specific binding pair member for the ligand immobilized on the reaction surface of the flow-through assay device. In a second embodiment, the target sequence is detected by hybridization of the ligand-containing probe to a complementary capture sequence immobilized on the reaction surface. In either embodiment the bound ligand-containing nucleic acid may be detected by binding the ligand to a labeled anti-ligand antibody or a labeled specific binding partner for the ligand. Alternatively, the target sequence may be detected in a sandwich assay format by hybridization with a ligand-containing probe and an immobilized capture sequence on the reaction surface, both probe and capture sequence being complementary to the target. The preferred ligand for incorporation into nucleic acids complementary to the target sequence is biotin.

## DESCRIPTION OF THE DRAWINGS

Fig. 1 is an illustration of one of the embodiments of the flow-through assay device of the invention.

Fig. 2 is a graphic depiction of the results of the experiments described in Example 3 for various sizes of capture and probe sequences. Fifty nanograms of each capture sequence and 100 ng of each probe were used in the experiment.

Fig. 3 is a graphic depiction of the results of the experiments described in Example 3 showing the effect of capture sequence size on signal intensity. One hundred ng of 24 and 50 bp capture sequences and 50 ng of 250 bp capture sequences were used in the experiment.

## DETAILED DESCRIPTION OF THE INVENTION

According to the invention, nucleic acid probes complementary to a desired target nucleic acid sequence are synthesized with incorporation of a ligand, using the target sequence as a template for synthesis. The ligand may serve as a means for capturing the probe on the reaction membrane of a flow-through assay device and/or as a means for detecting the presence of the probe on the reaction membrane.

The preferred flow-through membrane assay devices which are useful to perform the nucleic acid hybridization assays of the invention are essentially those known in the art for use in flow-through immunoassays. Exemplary devices for flow-through immunoassays suitable for adaptation to the invention are described in U.S. Patent No. 4,366,241, U.S. Patent No. 4,632,901, U.S. Patent No. 4,818,677 and U.S. Patent No. 4,920,046. The principles of operation of these devices remain the same when adapted to the inventive nucleic acid assay procedures, however, some modifications in the reagents and protocols are necessary to achieve specificity and sensitivity, as disclosed below.

The flow-through assay device includes a porous membrane on which the binding or hybridization reaction takes place as fluids flow through it. A variety of materials suitable for use as reaction membranes are known for particular assay applications. Nylon or nitrocellulose membranes are most often used for nucleic acid studies and are suitable for use in the assay of the invention. Nylon membranes such as the BIODYNE membranes available from Pall BioSupport Corporation (Glen Cove, N.Y.) are preferred because the process of immobilizing nucleic acids on the membrane by UV irradiation is simple to perform. Most preferred are BIODYNE B membranes in which the nylon membrane surface has been modified with cationic quarternary ammonium groups to allow rapid immobilization of nucleic acids without UV irradiation.

The target nucleic acid sequence is the oligonucleotide sequence to be detected. It is preferably a DNA oligonucleotide but may also be RNA. The target sequence may be any nucleic acid sequence suitable for use as a template for synthesis of a complementary probe as described below. Examples of target sequences include plasmids or portions thereof, genomic DNA sequences, DNA sequences derived from subcellular organelles, messenger RNAs and ribosomal RNAs, all of which may be isolated and prepared for synthesis of the complementary probe using methods known in the art.

To perform the assay, a sample containing or suspected of containing the target nucleic acid sequence is prepared, usually in solution, for synthesis of a ligand-containing probe which is complementary to the target sequence. A ligand is a molecule which specifically binds to a second molecule, i.e., it is a member of a specific binding pair. Antigens, antibodies, biotin, lectins and haptens are examples of ligands which bind specifically to antigens, avidin or streptavidin, carbohydrates and antibodies, respectively. Synthesis of the probe may be accomplished by target-specific cDNA or RNA synthetic methods which allow the incorporation of ligand-derivatized oligonucleotide precursors (deoxynucleoside triphosphates or dNTPs) into the complementary probe, e.g., polymerase chain reaction or other nucleic acid amplification methods which result in a defined probe sequence. Polymerase chain reaction (PCR) is the preferred means for synthesizing the probes. Preferably biotin-derivatized nucleotides are incorporated into the probe, but a variety of hapten ligands may also be incorporated, for example digoxigenin.

A tracer is used to detect the complementary probe. The tracer comprises a specific binding pair member for the ligand conjugated to a label. The specific binding, pair member may be an antibody which recognizes the ligand (e.g., anti-biotin) or a specific binding partner for the ligand (e.g., avidin or streptavidin for a biotin ligand). The label portion of the tracer is a detectable moiety which facilitates identification of the probe when it is immobilized on the reaction membrane of the flow-through assay device. The label may be directly detectable or it may be rendered detectable after further chemical reaction. For example, the label may be a radioisotope, a fluorescent dye or a visible colored dye, all of which are directly detectable visually or by instrumentation. Enzymes which are capable of reacting with additional chemical components to produce a colored product are examples of indirectly detectable labels, and include alkaline phosphatase and horse radish peroxidase. Liposomes encapsulating a visible colored dye and derivatized with the specific binding molecule are preferred labels. These labels and their use in assays are described in U.S. Patent No. 4,695,554, U. S. Patent No. 4,717,676 and U.S. Patent No. 4,708,933.

The ligand-containing probes complementary to the target sequence can be detected in a variety of ways. In one embodiment, an anti-ligand antibody (preferably anti-biotin) is nondiffusively immobilized on the reaction membrane of a flow-through assay device. This embodiment is useful for capture of ligand-containing single stranded or double stranded nucleic acids such as polymerase chain reaction products (single stranded or hybridized to the template nucleic acid) synthesized with a biotinylated deoxynucleoside triphosphate. It has been unexpectedly found that there unincorporated ligand-derivatized nucleic acid percursors do not interfere with the assay result, i.e., they do not appear to compete significantly with the probe for binding to the anti-biotin on the membrane. This embodiment, in which a specific binding partner or antiligand antibody is immobilized on the membrane for capture of the probe has the advantage of being independent of the specific target sequence being detected, as the anti-ligand or specific binding partner membrane can be used to capture any probe containing the ligand bound by the antibody or specific binding partner.

In an alternative preferred embodiment, the single stranded target sequence and/or its complementary strand, or a portion thereof, may be immobilized on the reaction membrane as a capture sequence and the probe synthesis reaction mixture applied such that the ligand-containing probe hybridizes to the capture sequence as it flows through the membrane. The capture sequence may be synthesized, generated by PCR or it may be a restriction fragment which includes the target sequence. Preferably the capture sequence will be a subset of the target sequence being used for synthesis of the complementary probe to reduce nonspecific hybridization of capture sequence and probe in the absence of the target sequence. If the probe is generated using PCR, the capture sequence also will preferably be derived from a portion of the target sequence which does not include sequences complementary to the primers used for amplification so that there will be no hybridization with unextended primers which may be present. The reaction membrane may also be blocked with protein, polynucleotides, polysaccharides, etc. to reduce nonspecific binding to the membrane.

The capture sequence immobilized on the membrane is denatured and nondiffusively bound to the membrane using appropriate materials and methods. These include heat or ultraviolet irradiation for fixation to amino-modified nylon membranes (e.g., BIODYNE B) or application to nitrocellulose. The capture sequence may be denatured by heating as well as chemical or enzymatic means. Studies were done to determine the maximum amount of capture sequence which could be retained by the reaction membrane. For this purpose, from 25 ng to 3 mg of a labeled 250 bp capture sequence was spotted on BIODYNE B membranes, followed by addition of all the solutions customarily used for the hybridization assay. The results demonstrated that between 500 ng and 750 ng of capture sequence the loss of capture sequence from the membrane begins to increase to over 25%. This suggests that the membrane becomes saturated with a 250 bp capture sequence at approximately 500 ng.

The probe synthesis reaction mixture, usually with a diluent, is applied to the immobilized capture sequence on the membrane such that, if a probe has been synthesized, hybridization between the complementary sequences occurs as the mixture flows through the membrane. Preferably, the mixture applied to the membrane includes components which slightly increase the viscosity to slow the flow of fluid through the membrane. For example, dextran sulfate (up to about 10%) and Denhardt's solution (about 1X, containing Ficoll and PVP) are suitable thickeners for this purpose. A washing step is usually included after hybridization to remove unbound probe sequences and reduce nonspecific binding to the membrane. The hybridized probe may then be detected on the membrane using a tracer as previously described. Preferably, the ligand contained in the probe is biotin and the tracer is an anti-biotin derivatized liposome encapsulating a dye which produces an area of visible color on the membrane if probe has hybridized to the capture sequence. Hybridization of probe on the membrane indicates that the target sequence is present in the sample used for synthesis of the probe.

In another alternative embodiment, the assay may be performed as a flow-through sandwich assay. In this embodiment, the capture sequence immobilized on the reaction membrane will be complementary to a portion of the target sequence. The probe is synthesized to be complementary to a second portion of the target sequence such that hybridization of the probe to the target sequence will not interfere with hybridization of the capture sequence to the target sequence. In the sandwich assay, the capture sequence and the probe are hybridized to the target sequence. The capture sequence immobilizes the hybridized capture/target/probe complex on the membrane. The probe and target sequence may be hybridized in solution prior to application to the capture sequence on the membrane. However, it is preferred that the target sequence be hybridized to the capture sequence, followed by hybridization of the probe to the capture sequence/target sequence hybrid, i.e., in a stepwise hybridization. The stepwise method is faster because the slower solution hybridization is eliminated. The immobilized probe may then be detected as previously described.

For convenience, the devices and/or reagents for the assay may be packaged in the form of a kit. Such a kit may contain several or all of the following components of the assay: 1) a vial of ligand-derivatized nucleic acid precursors, 2) reagents for oligonucleotide probe synthesis appropriate for the selected method, 3) a flow-through assay device having the appropriate ligand binding partner or capture sequence immobilized on the reaction membrane, 4) wash reagents, 5) a tracer and 6) reagents for detecting the label of the tracer if it is not directly detectable. Alternatively, presynthesized probes with the incorporated ligand may be provided for use in a particular assay application, e.g., detection of Neisseria.

The following experimental examples are intended to illustrate specific embodiments of the invention and are not intended to be limiting. Modifications and variations of the invention will be apparent to those skilled in the art without the exercise of inventive skill and without departing from the spirit and scope of the invention as defined by the appended claims.

## EXAMPLE 1

Target and probe sequences were generated by PCR. Target fragments were amplified using the following conditions: 62.5 ml water, 10.0 ml 10X AMPLITAQ buffer (Perkin-Elmer Cetus, Norwalk, CT), 16 ml 1.25 mM dNTP's, 5.0 ml 20 mM forward primer, 5.0 ml 20 mM reverse primer, 1-20 ng template nucleic acid in 1.0 ml total volume and 0.5 ml AMPLITAQ DNA polymerase (5 units/ml, Perkin-Elmer Cetus). AMPLITAQ 10X buffer contained 100 mM Tris-Cl pH 8.3, 500 mM KCl, 15 mM $MgCl_2$ and 0.01% gelatin. Probe fragments were amplified using the following conditions: 57.7 ml water, 10.0 ml 10X AMPLITAQ buffer, 16 ml dNTP's (1.25mM A,C,G; 0.94 mM T), 5.0 ml 1 mM biotin-16-dUTP, 5.0 ml 20 mM forward primer, 5.0 ml 20 mM reverse primer, 1-20 ng template nucleic acid in 1.0 ml total volume and 0.5 ml AMPLITAQ DNA polymerase (5 units/ml). The template was a BLUESCRIPT plasmid (Stratagene. La Jolla, CA) containing a 316 bp PstI/PvuII fragment from the 5' portion of the rubella E1 gene (D. M. Clarke, et al. Nucleic Acids Res. 15(7):3041) inserted into the PstI/SmaI site.

PCR amplification reactions were typically run for about 35 cycles, denaturing at 95°C for 2 min., annealing at 50°C for 2 min. and elongating at 72°C for 3 min. at each cycle. Alter amplification, 250 bp capture and probe sequences were purified by HPLC on a Perkin-Elmer PE TSK nonporous column to remove primers and unincorporated nucleotides, according to the manufacturer's protocol. This step allowewd the DNA to be precisely quantitated. Capture DNA was spotted onto the membranes in 1 ml of 10 mM Tris-Hcl pH 7.5, 1 mM EDTA (TE buffer). Varying amounts of the capture sequence were applied to the membrane to determine the optimal concentration for capture (50 ng, 10 ng and 5 ng).

EXAMPLE 2

Membranes prepared by applying 2 ml of DNA to 3 mm BIODYNE B membranes were wet with 0.2 M NaOH and crosslinked with ultraviolet light using a Stratagene STRATALINKER at a setting of 1200 mjoules. The membranes were then neutralized by washing with 100 mM sodium phosphate, pH 8.0, 10 mM EDTA. The membranes were baked at 45°C for 30 minutes and stored with a desiccant if not immediately used. The assay was performed by laying the membrane containing the capture DNA on an absorbant pad as shown in Fig. 1. The absorbant pad directs the flow of fluids through the nylon membrane. The absorbant pad comprises a top polycarbonate layer (1 mm pore size). a polyester layer in contact with the polycarbonate layer and a layer of absorbent cellulose material in contact with the polyester layer. The layers of the absorbant pad may be sewn together.

Two to five microliters of the biotinylated probe were added to 40 ml of Hybridization Buffer I (Hyb I) comprising 48% formamide, 0.72 M sodium (4.8X SSC), 20 mM Tris-Cl pH 7.6, 1X Denhardt's solution, 10% dextran sulfate and 0.1% SDS. The probe solution was heated at 70°C for 5 minutes. The heated probe was immediately added dropwise to the nylon membrane. All subsequently added solutions were also added dropwise. When all of the hybridization solution had flowed through the membrane, two wash solutions were applied. The first (150 ml of 2X SSC containing 0.1% SDS) was applied and allowed to flow completely through, then the second wash solution was added (150ml 0.2X SSC/0.1% SDS). After washing, one hundred fifty microliters of a blocking solution (3% BSA (Fraction V), 2% blotto) were added to the membrane. The hybridized probe was then detected with 150 ml of a suspension of anti-biotin conjugated liposomes containing sulforhodamine B dye. Liposomes specifically bound to the membrane produced a red dot in the position where the capture DNA had been applied. Loosely associated liposomes on the membrane around the red dot produced a faint pink color which was cleared in a final wash with 150 ml of 1 M guanidine, pH 7.0.

Assay results are shown in the following table. In this assay it was found that 50 ng of capture DNA was needed to detect 5 ng of the probe. When smaller amounts of capture DNA were used the assay was less sensitive.

| CAPTURE DNA | | | |
|---|---|---|---|
| PROBE DNA | 5ng | 10 ng | 50 ng |
| 350 ng | - | + | + |
| 70 ng | - | + | + |
| 35 ng | - | + | + |
| 10 ng | - | - | + |

The hybridization reaction was also shown to be specific. A 500 bp nucleic acid fragment of lambda DNA was amplified and biotin-labeled for use as a nonhomologous control capture sequence (Perkin/Elmer Cetus GENEAMP control fragment). Fifty nanograms were applied to a membrane and tested against the 250 bp target sequence. There was no visible reaction. A strong positive reaction was seen when a flow-through assay was performed using the amplified lambda fragment as both the capture and target sequence, confirming that the fragment would hybridize to itself.

EXAMPLE 3

In this experiment the sizes of both the capture sequence and the probe sequence were varied. The DNA preparations and other procedures were as described in Example 1. Fifty nanograms of each capture sequence (150 bp, 250 bp, 850 bp and 1,650 bp) were spotted on a nylon membrane and hybridized with a panel of probes of the same size. To perform the assay, 5 ml (100 ng) of biotinylated probe added to 40 ml of Hyb I and heated at 70°C for 5 minutes. The mixture was added dropwise to the membrane and the assay performed as described in Example 2. The intensity of the reaction was quantitated using a Gretag Model D183 reflectometer (Gretag, Ltd., Regensdorf, Switzerland). The results are shown in Fig. 2. In all instances probe hybridization and the resulting signal could be detected by the reflectometer. The largest probe (1,650 bp) gave the most intense signal with all sizes of capture DNAs. In contrast, the other probes in general gave signals of decreasing intensity with decreasing probe sequence size. The specificity of the assay using the different size fragments was tested by using nonhomologous capture and probe fragments and in all cases the readings were similar to background levels.

The nonhomologous probe did produce a very weak positive reaction with the largest capture fragment (1,650 bp). This may be due to a small amount of nonspecific hybridization due to the relatively large size of the capture fragment or to partial pore blockage in the membrane interfering with free flow of the probe through the membrane.

In a similar experiment, the capture sequences of 50 bp and 24 bp were found to be capable of capturing the 850 bp, 450 bp, 250 bp and 150 bp probes. However, as shown in Fig. 3, the intensity of the signal was reduced as compared to the assay using larger capture sequences. In this case, 100 ng of 24 bp or 50 bp capture DNA was required to detect 200 ng of probe. Even with this increase in the amount of DNA, the intensities were less than those seen when smaller amounts of the larger fragments were used (i.e., 50 ng of the 250 bp capture fragment).

EXAMPLE 4

Neisseria gonorrhea was used as a model system to demonstrate the utility of the inventive method for detecting a specific nucleic acid fragment in cells. Primers were selected and synthesized to amplify an approximately 281 bp portion of the gonococcal H.8 gene from nucleotides 53 to 320 as reported by Woods et al. (1989) Molecular Microbiology 3 (1): 43-48. This gene is highly conserved among the pathogenic Neisseria species. Amplified probes were synthesized using the polymerase chain reaction as previously described.

For the flow-through hybridization assay, Neisseria gonorrhea strain GC5766 was grown in liquid culture. Ten fold serial dilutions were made in 200 ml of 2 mM Tris-Cl, pH 8.5, 2 mM EDTA, 1% Triton X-100 to obtain $10^6$ to $10^2$ organisms per ml. Each dilution was heated to 95°C for 10 minutes. Twenty five microliters of each dilution were used as templates in PCR reactions. The 281 bp fragment of the H.8 gene was amplified, incorporating biotin-16 UTP as described above. Thirty microliter aliquots of the PCR reactions added to 100 ml of Hyb I were tested in the flow-through assay after 40 cycles and 50 cycles of amplification. The homologous unlabeled capture fragment on the membrane was generated by PCR using purified GC5766 genomic DNA as the template. As few as 250 organisms could be detected at 40 cycles. Aliquots of the amplification reaction were also run on an agarose gel for visualization of the reaction products. The gel was significantly less sensitive than the hybridization assay. With an additional 10 cycles of amplification (50 cycles) as few as 2.5 cells could be detected on flow-through hybridization, but were very difficult to visualize on the gel.

EXAMPLE 5

The flow-through hybridization assay may also be done in a sandwich assay format. A small 150 bp fragment of the rubella E1 gene was used as the capture sequence for a 1,650 bp target sequence. A probe was prepared by amplification of a 400 bp portion of the 1,650 bp target sequence which would not hybridize with the capture sequence, incorporating biotin-16-UTP as described above.

The 150 bp capture sequence was spotted on BIODYNE B membranes at 50 ng and 10 ng in 2 ml. The assay was performed essentially as in Example 2. Five microliters of target sequence, purified and diluted in TE buffer, were mixed with 45 ml of Hyb I and heated at 70°C for 5 min. The prepared target DNA was then applied to the nylon membrane and allowed to flow through. The membrane was washed with 45 ml of 0.2X SSC followed by 45 ml of 2X SSC and 150 ml of 100 mM PBS pH 8.0. Probe (92 ng) in 5 ml was added to 45 ml of hybridization buffer, heated at 70°C for 5 minutes and allowed to flow through the membrane. The membrane was washed as before with 150 ml of each of the SSC solutions and blocked with 150 ml of Blotto/BSA solution as described above. Rabbit anti-biotin liposomes encapsulating sulforhodamine (150 ml of a 1:3 dilution in 1% BLOTTO) were used to detect hybridized probe on the membrane, followed by addition of 2 drops of 2 M urea.

Several concentrations of probe and capture DNA were evaluated. It was found that using 92 ng of probe and 50 ng of capture DNA (150mer) the system could detect 4 ng of 1,650 bp target fragment. Using these same probe and capture fragments and a nonhomologous target (plasmid pMSG, Pharmacia) there was no detectable reaction with 25 ng of the pMSG target, demonstrating that the reaction was specific.

## Claims

**1.** A method for detecting a target nucleic acid sequence comprising:

a) synthesizing a complementary probe using the target sequence as a template, the probe including a ligand;

b) applying the probe to a flow-through assay device having a capture sequence immobilized on a porous reaction membrane such that the probe flows through the reaction membrane substantially immediately and hybridizes to the capture sequence;

c) contacting the membrane with a tracer comprising a specific binding molecule for the ligand conjugated to a detectable label, such that the tracer binds to the ligand, and;

d) detecting the tracer on the membrane as an indication of the presence of the target sequence.

**2.** The method of Claim 1 wherein the probe is synthesized to include a biotin-derivatized nucleotide and the biotin binds to a tracer comprising an anti-biotin antibody.

**3.** The method of Claim 2 wherein the biotin binds to a tracer comprising an anti-biotin liposome encapsulating a dye.

**4.** A method for detecting a target nucleic acid sequence comprising:

a) synthesizing a probe complementary to a first portion of the target sequence using the first portion of the target sequence as a template, the probe including a ligand;

b) applying the target sequence and the probe to a flow-through assay device having a capture sequence immobilized on a porous reaction membrane, the capture sequence being complementary to a second portion of the target sequence;

c) allowing the target sequence and the probe to flow through the reaction membrane such that the target sequence hybridizes to the capture sequence and the probe hybridizes to the target sequence;

d) contacting the membrane with a tracer comprising a specific binding molecule for the ligand conjugated to a detectable label such that the tracer binds to the ligand, and;

e) detecting the tracer on the membrane as an indication of the presence of the target sequence.

**5.** The method of Claim 4 wherein the probe is synthesized to include a biotin-derivatized nucleotide and the biotin binds to a tracer comprising an anti-biotin antibody.

**6.** The method of Claim 5 wherein the target sequence and the probe are hybridized prior to the application to the reaction membrane.

**7.** The method of Claim 5 wherein the target sequence is applied to the membrane prior to applying the probe to the membrane.

**8.** The method of Claim 5 wherein the biotin binds to a tracer comprising anti-biotin derivatized liposomes encapsulating a dye.

**9.** A method for detecting a target nucleic acid sequence comprising:

a) synthesizing a complementary probe using the target sequence as a template, the probe including a ligand;

b) applying the probe to a flow-through assay device having an anti-ligand antibody immobilized on a porous reaction membrane such that the probes flows through the reaction membrane substantially immediately and binds to the anti-ligand antibody;

c) contacting the membrane with a tracer comprising a specific binding molecule for the ligand conjugated to a detectable label such that the tracer binds to the ligand, and;
d) detecting the tracer on the membrane as an indication of the presence of the target sequence.

**10.** The method of Claim 9 wherein the probe is synthesized to include a biotin-derivatized nucleotide, the probe binds to an anti-biotin antibody immobilized on the reaction membrane and the biotin binds to a tracer comprising an anti-biotin antibody.

FIG—1

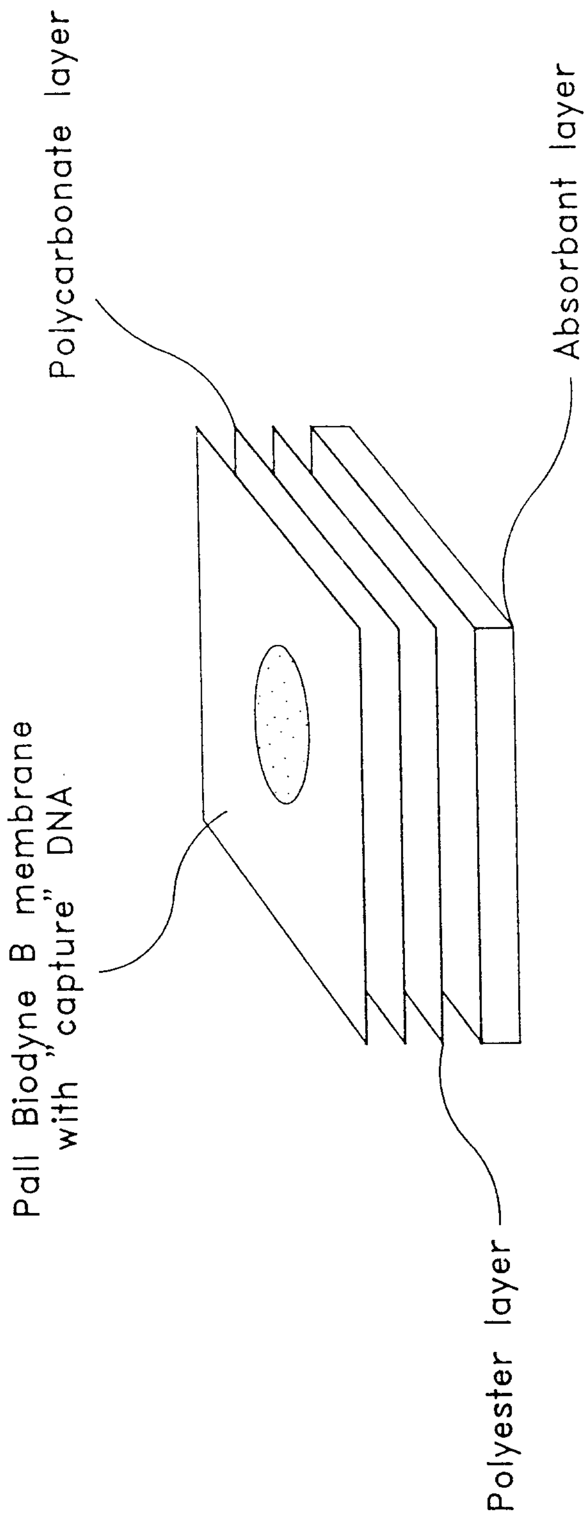
Polycarbonate layer
Absorbant layer
Pall Biodyne B membrane with "capture" DNA
Polyester layer

FIG-2

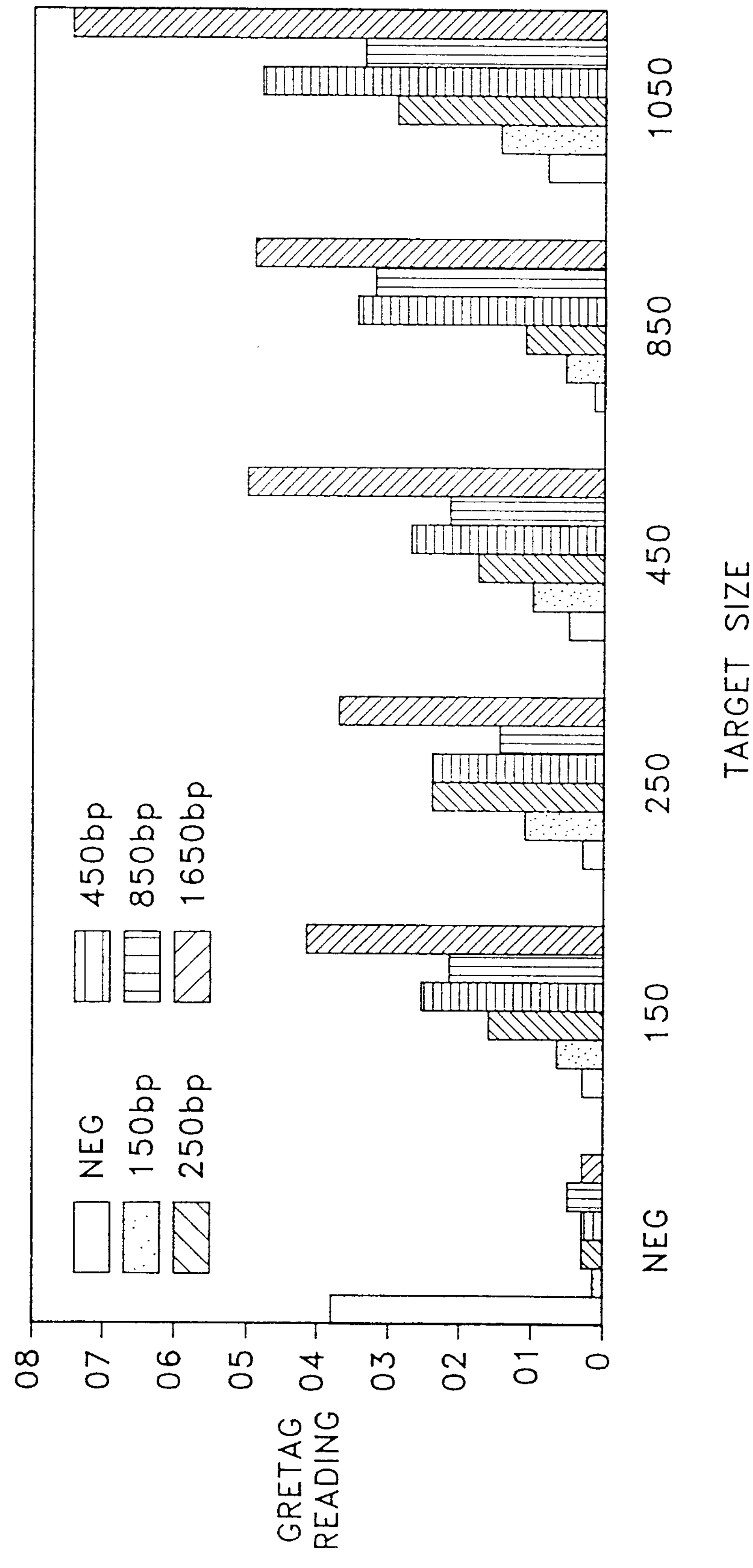
GRETAG READING
0
01
02
03
04
05
06
07
08
TARGET SIZE
NEG
150
250
450
850
1050
NEG
150bp
250bp
450bp
850bp
1650bp

FIG-3

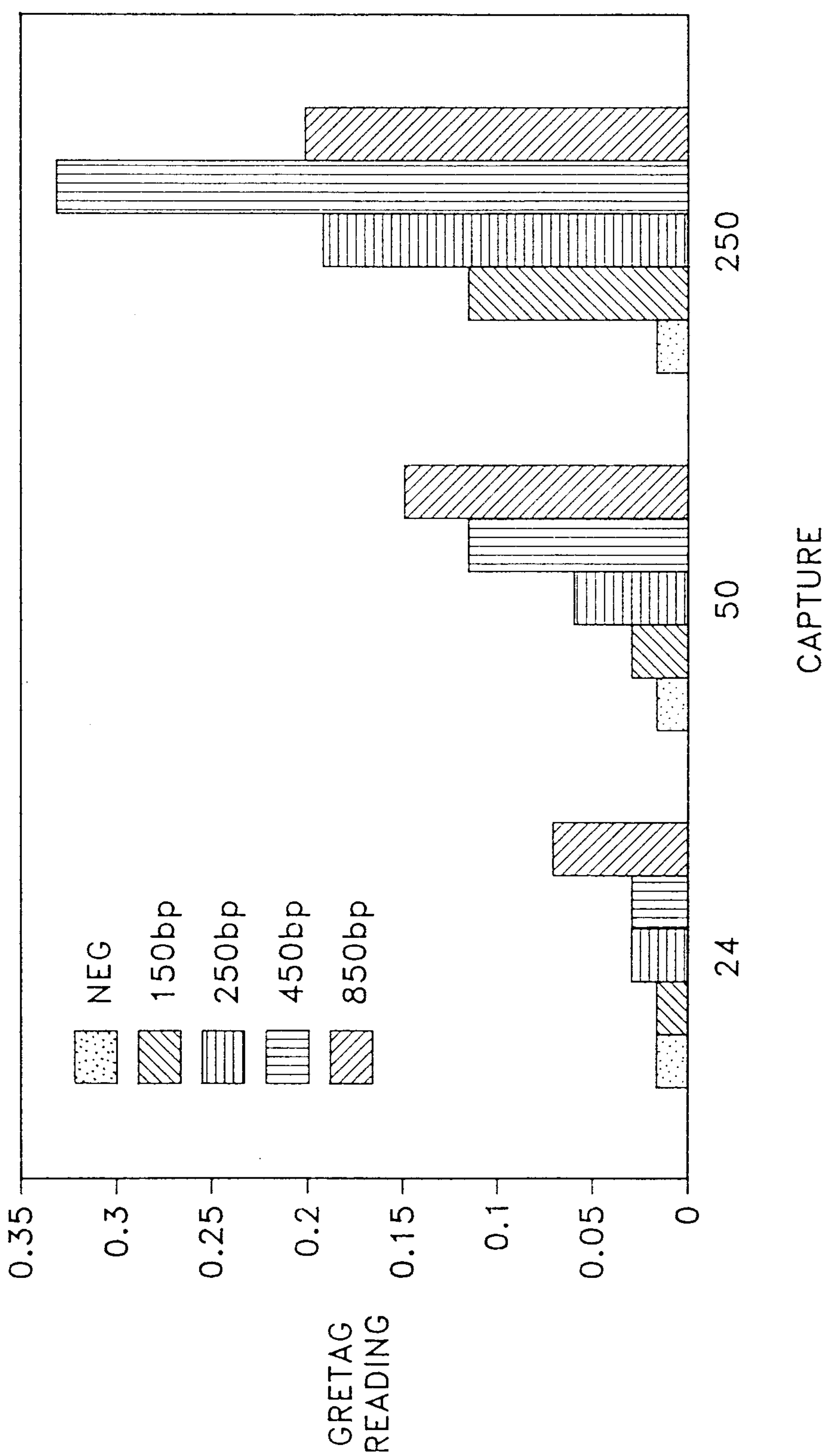
GRETAG
READING
0.35
0.3
0.25
0.2
0.15
0.1
0.05
0
NEG
150bp
250bp
450bp
850bp
24
50
250
CAPTURE

European Patent Office

# EUROPEAN SEARCH REPORT

Application Number
EP 93 12 0394

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 458 231 (BECTON DICKINSON AND CO.)<br>* the whole document *<br>--- | 1-9 | C12Q1/68<br>C12P19/34<br>G01N33/543 |
| X | EP-A-0 302 673 (BECTON DICKINSON AND CO.)<br>* the whole document *<br>--- | 1-9 | |
| X | EP-A-0 335 244 (ABBOT LABORATORIES)<br>* the whole document *<br>--- | 1-9 | |
| A | EP-A-0 455 905 (MICROPROBE CORP.)<br>* page 8; examples 3-5 *<br>--- | 1-9 | |
| X<br>A | WO-A-90 04786 (MOLECULAR DEVICES CORP.)<br>* examples 3-6 *<br>--- | 9<br>1 | |
| A | WO-A-92 16659 (EASTMAN KODAK CO.)<br>* the whole document *<br>----- | 1-9 | |

TECHNICAL FIELDS SEARCHED (Int.Cl.5)

C12Q
G01N

The present search report has been drawn up for all claims

| Place of search | Date of completion of the search | Examiner |
|---|---|---|
| THE HAGUE | 15 April 1994 | Osborne, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document